# FASCICULE DE BREVET EUROPEEN

(11) **EP 1 660 845 B1**
(45) Date de publication et mention de la délivrance du brevet: **20.05.2015**
(21) Numéro de dépôt: 04767675.4
(22) Date de dépôt: 13.07.2004
(51) Int. Cl.: G06M 1/04, A61M 15/00

(54) **INDICATEUR DE DOSES AMELIORE POUR DISPOSITIF DE DISTRIBUTION DE PRODUIT FLUIDE**
VERBESSERTER DOSISINDIKATOR FÜR EINE FLUIDPRODUKT-ABGABEEINRICHTUNG
IMPROVED DOSE INDICATOR FOR FLUID PRODUCT DISPENSING DEVICE

(30) Priorité: 18.07.2003 FR 0308834
(43) Date de publication de la demande: 31.05.2006
(73) Titulaire: Aptar France SAS, 27110 Le Neubourg (FR)
(72) Inventeur: STRADELLA, Fabio, I-16032 Camogli (IT); STRADELLA, Giuseppe, I-16032 Camogli (IT)
(74) Mandataire: CAPRI
(86) Numéro de dépôt international: PCT/FR2004/001846
(87) Numéro de publication internationale: WO 2005/017463

(56) Documents cités:
- WO-A-00/09187
- WO-A-01/37909
- GB-A- 1 336 014
- US-A- 5 349 945

## Description

La présente invention concerne un indicateur de doses, ainsi qu'un dispositif de distribution de produit fluide comportant un tel indicateur.

Dans le domaine des dispositifs de distribution de produit fluide destinés à distribuer plusieurs doses, et en particulier dans le domaine des pulvérisateurs, de nombreux systèmes destinés à indiquer le nombre de doses distribuées ou le nombre de doses restant à distribuer ont été développés.

La plupart de ces systèmes présentent de nombreux inconvénients. Ainsi, ils sont généralement conçus aux moyens de plusieurs roues dentées formant des engrenages, dont le nombre dépend de la quantité de doses à compter. Par conséquent, ces compteurs ou indicateurs peuvent devenir très complexes, encombrants, et donc coûteux à fabriquer et à assembler. D'autre part, l'indication est généralement donnée par des chiffres, qui sont souvent difficiles à lire pour l'utilisateur, en particulier lorsque les dispositifs de distribution sont destinés à distribuer un grand nombre de doses, par exemple jusqu'à 200 doses. De même, tous les systèmes de compteurs ou d'indication de doses actuels ne sont pas utilisables par les personnes ayant des problèmes de vue, et notamment par les aveugles. Un autre inconvénient majeur réside dans le fait que les compteurs existants nécessitent généralement une procédure d'assemblage du dispositif de distribution qui est modifiée de par la présence du compteur, et qui diffère donc de la procédure d'assemblage habituelle. Ceci augmente la complexité du dispositif, et implique par conséquent un coût supérieur.

D'autre part, une exigence de sécurité très important est d'éviter tout risque de sous comptage, c'est-à-dire de ne pas compter une distribution totale ou partielle de produit. Pour éviter ce risque, il est nécessaire que l'actionnement du compteur soit réalisé pendant la course de l'organe de distribution, en particulier de la soupape de valve, qui se produit avant le début de l'expulsion du produit. La longueur de cette course initiale est généralement très courte, typiquement de l'ordre de 1 à 1,5 mm, et les diverses tolérances de dimension du dispositif réduisent celle-ci à quelques dixièmes de millimètres. Une course d'actionnement aussi courte rend difficile l'actionnement du compteur et peut impliquer l'utilisation de mécanismes complexes pour garantir un comptage fonctionnel. Le document WO 00/09187 décrit un dispositif de l'art antérieur.

La présente invention a pour but de fournir un indicateur de doses destiné à un dispositif de distribution de produit fluide, qui ne reproduit pas les inconvénients susmentionnés.

En particulier, la présente invention a pour but de fournir un indicateur de doses qui est simple et peu coûteux à fabriquer et à assembler, et qui peut notamment être appliqué à tous les dispositifs de distribution de produit fluide existants sans impliquer une modification de la procédure d'assemblage.

La présente invention a aussi pour but de fournir un indicateur de doses qui est de petite dimension, indépendamment du nombre de doses contenu dans le dispositif de distribution.

La présente invention a également pour but de fournir un indicateur de doses qui forme une unité complète et séparée, et qui comprend notamment les moyens d'actionnement de l'indicateur.

La présente invention a aussi pour but de fournir un indicateur de doses qui soit facile à lire pour l'utilisateur, et qui peut aussi être utilisé par des personnes ayant des problèmes de vue, et notamment par les aveugles.

La présente invention a encore pour but de fournir un indicateur de doses qui évite tout risque de sous comptage (non prise en compte d'une dose distribuée). Plus particulièrement, la présente invention à pour but de fournir un indicateur de doses qui compte dès le début de la course d'actionnement du dispositif de distribution auquel il est associé, même si cette course est très courte.

La présente invention a donc pour objet un indicateur de doses pour dispositif de distribution de produit fluide tel que décrit dans la revendication 1. Des variantes avantageuses sont décrites dans les revendications dépendantes.

La présente invention a aussi pour objet un dispositif de distribution de produit fluide, comportant un réservoir de produit et un organe de distribution, tel qu'une pompe ou une valve, monté sur ledit réservoir, ainsi qu'un indicateur de doses tel que défini ci-dessus.

Avantageusement, l'indicateur de dose est actionné par une partie du dispositif de distribution qui est déplacée lors de l'actionnement du dispositif, et qui coopère avec un élément de transmission dudit indicateur.

D'autres caractéristiques et avantages de la présente invention apparaîtront plus clairement au cours de la description détaillée suivante d'un mode de réalisation particulier de celle-ci, faite en référence aux dessins joints, donnés à titre d'exemples non limitatifs, et sur lesquels,
- la figure 1 est une vue schématique de côté partiellement découpée d'un dispositif de distribution de produit fluide comportant un indicateur de doses selon un mode de réalisation avantageux de la présente invention,
- la figure 2 est une vue de face, similaire à celle de la figure 1,
- la figure 3 est une vue éclatée d'un indicateur de doses selon un mode de réalisation avantageux de l'invention,
- la figure 4 est une vue similaire à celle de la figure 3, prise selon un autre angle de vue,
- la figure 5 est une vue schématique des moyens d'actionnement de l'indicateur des figures 3 et 4, en position de repos,
- la figure 6 est une vue similaire à celle de la figure 5, en début d'actionnement, et
- la figure 7 est une vue similaire à celle des figures 5 et 6, en fin d'actionnement.

L'indicateur de doses A de la présente invention s'applique à tous types de dispositifs de distribution de produit fluide. Il s'applique toutefois plus particulièrement aux dispositifs de pulvérisation, et avantageusement aux dispositifs aérosols comportant une valve doseuse montée sur un récipient contenant un produit et un gaz propulseur.

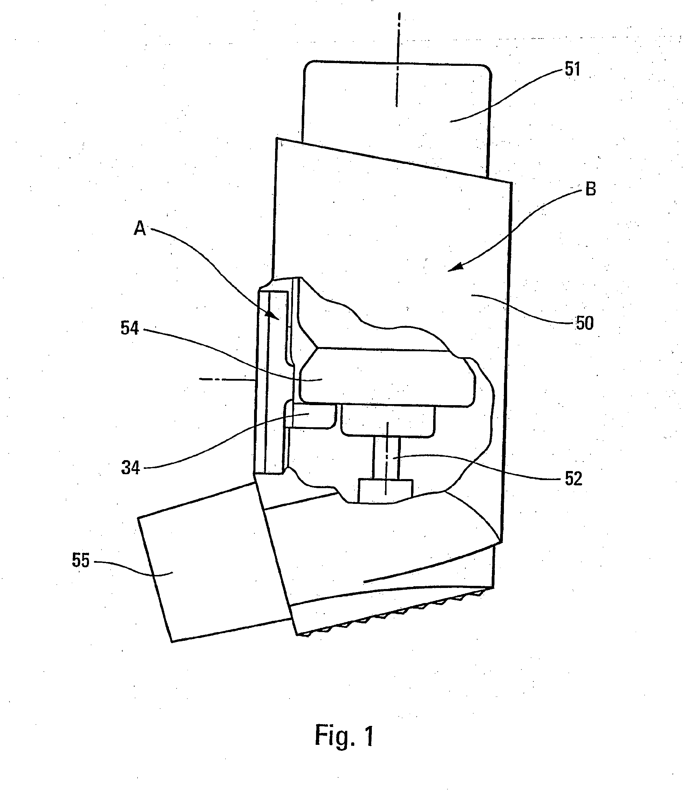

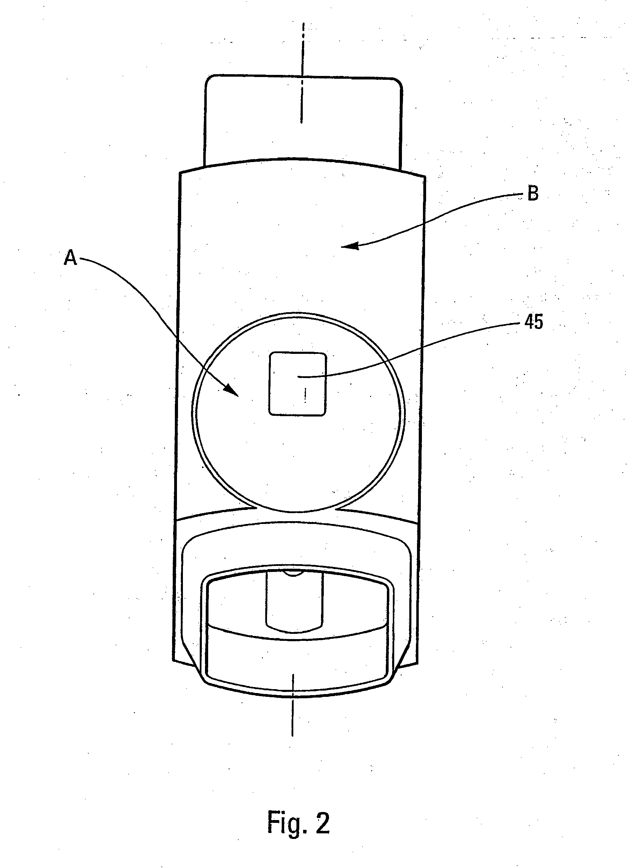

Les figures 1 et 2 représentent schématiquement un dispositif de distribution B auquel l'indicateur de doses A de la présente invention est particulièrement adapté. Ce dispositif comporte un corps 50 et un réservoir 51 sur lequel est assemblé une valve doseuse 52. L'actionnement du dispositif B étant obtenu par déplacement axial du réservoir 51 à l'intérieur du corps 50, ce déplacement entraînant une compression de la soupape de la valve 52 ce qui provoque l'expulsion d'une dose de produit à travers un orifice buccal 55. Bien entendu, la présente invention s'applique également à d'autres types de dispositifs de distribution, et notamment des dispositifs de pulvérisation du type nasal, ou des dispositifs comportant une pompe en lieu et place de la valve.

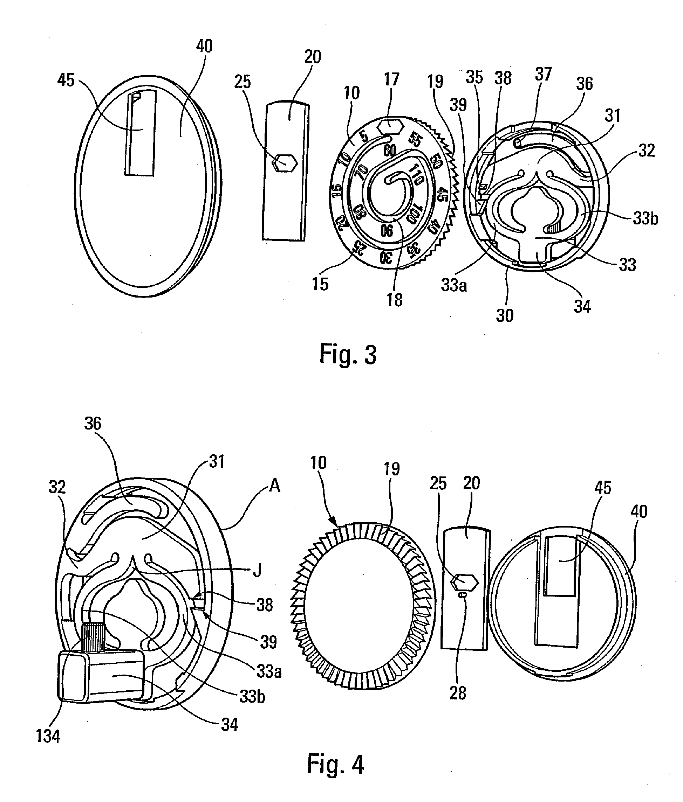

Les figures 3 à 7 représentent un indicateur de doses A qui peut notamment être utilisé avec un dispositif de distribution de produit fluide B décrit ci-dessus. Cet indicateur de doses comprend au moins un moyen de comptage rotatif qui, dans l'exemple représenté, est formé par une roue de comptage rotative 10, de préférence réalisée sous la forme d'un disque rotatif, adaptée à se déplacer en rotation autour d'un axe de rotation sensiblement perpendiculaire audit disque 10. Ce disque rotatif 10 est de préférence mince et pourvu d'un profil creux 18, qui peut avantageusement être réalisé au moyen d'une nervure ou rainure. Le disque 10 comporte en outre avantageusement une denture 19, de préférence réalisée sur sa périphérie, ladite denture 19 étant adaptée à coopérer avec des moyens d'actionnement qui sont adaptés à faire tourner ledit disque 10, et qui seront décrits plus amplement ci-après. Le disque ou roue de comptage 10 comporte également des moyens d'indication 15, qui peuvent être des nombres et/ou des symboles, et qui sont destinés à indiquer le nombre de doses distribuées ou restant à distribuer. Ces moyens d'indication 15 suivent avantageusement au moins partiellement ledit profil creux 18.

L'indicateur A représenté sur les figures peut aussi comporter avantageusement un organe translatif 20, adapté à se déplacer en translation. Cet organe translatif 20 comporte une projection 28, ou tout autre moyen équivalent, qui coopère avec ledit profil creux 18 du disque rotatif 10. Cet organe translatif 20 est de préférence réalisé sous la forme d'une plaque mince, et comporte une ouverture de visualisation 25 destinée à coopérer avec les moyens d'indication 15 du disque rotatif 10.

Selon la forme du profil creux 18, une rotation de la roue de comptage 10 peut entraîner une translation de l'organe translatif 20. Avantageusement, le profil 18 est réalisé de telle sorte que l'indication est progressive et non régulière. Par exemple, l'indicateur des figures 3 et 4 peut compter environ 120 doses, les 50 dernières étant affichées par intervalle de 5 dans l'ouverture de visualisation 25 de l'organe translatif 20, alors que les premières sont indiquées par intervalle de 10. Dans cet exemple, le profil creux 18 est d'abord en spirale, au centre du disque 10, de sorte que chaque rotation dudit disque 10 entraîne une translation dudit organe translatif 20. Lorsqu'il ne reste que 50 doses à distribuer, le profil 18 devient cylindrique, de sorte que les rotations suivantes du disque 10 ne déplacent plus l'organe translatif 20. Les chiffres d'indication 18 s'affichent alors dans l'ouverture de visualisation 25 au fur et à mesure de l'actionnement du dispositif. Après la dernière dose, un symbole spécifique 17 peut indiquer qu'il ne reste aucune dose à distribuer. D'autres progressions sont aussi envisageables.

Avantageusement, la roue de comptage 10 et l'organe translatif 20 sont disposés dans un couvercle 40, qui est de préférence également de structure mince, et qui comporte une fenêtre de visualisation 45, coopérant avec l'ouverture de visualisation 25 de l'organe translatif 20 pour permettre à l'utilisateur de visualiser les moyens d'indication 15 de la route de comptage 10

L'actionnement de l'indicateur A, et en particulier la rotation de la roue de comptage rotative 10 peut avantageusement être réalisé par des moyens d'actionnement intégrés dans ledit indicateur A. Ces moyens d'actionnement peuvent avantageusement comporter un élément d'entraînement 31 réalisé sous la forme d'une patte flexible, solidaire d'une bague 30 qui entoure ladite denture 19 du disque rotatif 10. Cette patte flexible 31 est adaptée à coopérer avec ladite denture 19 à chaque fois qu'une dose est distribuée, de préférence au moyen d'un organe d'actionnement 35, tel qu'une dent. Avantageusement, on prévoit des moyens anti-retour 36, 37 pour empêcher ledit disque rotatif 10 de tourner dans le sens inverse à celui qui lui est donné par la patte flexible 31 lors de l'actionnement. Ces moyens anti-retour peuvent comporter une patte flexible 36 supportant une dent anti-retour 37 coopérant avec la denture 19.

Les moyens d'actionnement comportent également un élément de transmission 34 qui est adapté à coopérer avec le dispositif de distribution de produit fluide B, à chaque actionnement de celui-ci, ledit élément de transmission 34 coopérant d'autre part avec ladite patte flexible 31 pour faire tourner ledit disque rotatif 10. En particulier, comme visible notamment sur la figure 1, ledit élément de transmission 34 est un épaulement solidaire de la patte flexible 31 et qui coopère avec une partie 54 du dispositif de distribution de produit fluide B qui est mobile pendant l'actionnement. Dans l'exemple représenté, il s'agit de la bague de fixation 54 de la valve doseuse 52 sur le réservoir 51. Bien entendu, et plus généralement, toute partie qui se déplace lors de l'actionnement du dispositif B est adaptée à coopérer avec l'épaulement 34 pour actionner l'indicateur de doses A. Avantageusement, l'élément de transmission 34 peut comporter une cheville ajustable 134 permettant de compenser les tolérances de fabrication lors de l'assemblage du dispositif de distribution, ainsi que des courses d'actionnement variables, pour prédéterminer la course d'actionnement de l'indicateur A.

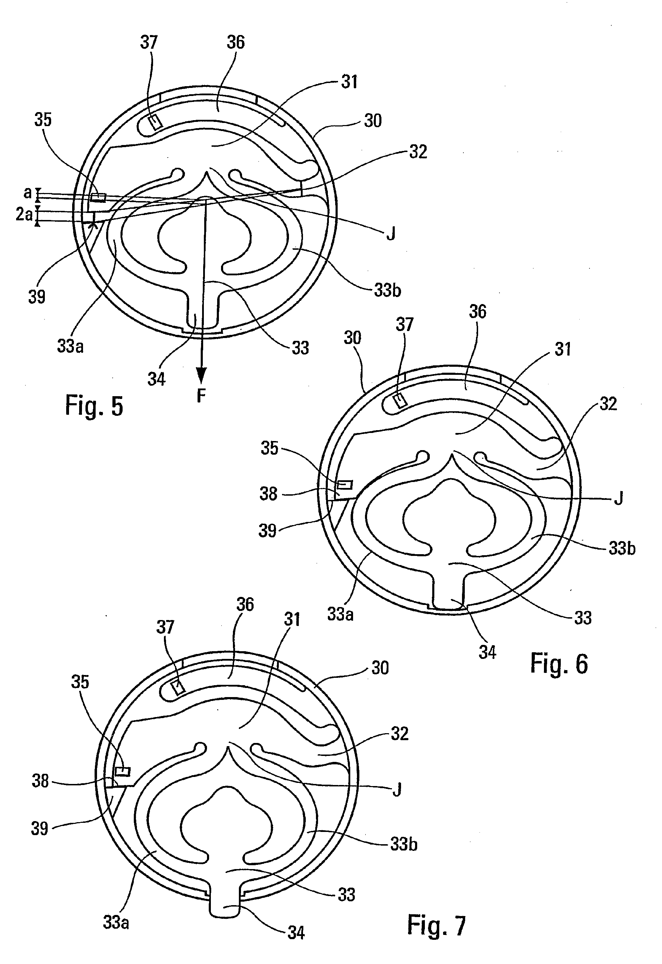

En référence aux figures 3 à 7, la patte flexible 31 peut être pourvue de deux parties flexibles 32 et 33 de flexibilité différente, la première partie 32 étant plus flexible que la seconde partie 33. La seconde partie de patte 33 supporte ledit épaulement 34, et lorsque le dispositif de pulvérisation B est actionné, la bague de fixation 54 du réservoir entraîne d'abord la partie plus flexible 32 du bras 31 à se fléchir parallèlement au disque rotatif 10, ce qui provoque la rotation dudit disque 10 au moyen de la dent d'actionnement 35 qui coopère avec la denture 19. La patte flexible 31 comporte avantageusement un élément de blocage 38 adapté à coopérer avec une butée saillante 39 solidaire de la bague 30. La distance radiale entre l'élément de blocage 38 et la butée 39 correspond avantageusement à une dent de la denture 19. Ainsi, pendant actionnement, l'épaulement 34 est déplacé (vers le bas sur les figures) par le dispositif de distribution B, et la partie de bras plus flexible 32 se fléchit (également vers le bas sur les figures), jusqu'à ce que l'élément de blocage 38 contacte la butée 39, comme visible sur la figure 6. Ceci entraîne la rotation sur l'équivalent d'une dent de la roue de comptage 10. La partie de bras plus flexible 32 est alors bloquée, et une poursuite de la course d'actionnement du dispositif de distribution B est possible par la flexion de la partie de bras moins flexible 33, comme visible sur la figure 7. De cette manière, on permet un actionnement de l'indicateur de doses dans la première partie de ladite course d'actionnement. Ceci élimine tout risque de non comptage d'une dose distribuée (partiellement ou totalement) en cas d'actionnement partiel du dispositif de distribution B, tout en permettant une poursuite de la course d'actionnement après comptage. La butée 39 et les moyens anti-retour 36, 37 assurent que chaque dose n'est comptée qu'une seule fois.

Selon l'invention, il est prévu des moyens d'amplification adaptés à amplifier le déplacement de l'élément de transmission 34 au tout début de la course d'actionnement, pour que le déplacement de l'organe d'actionnement (dent) 35 soit supérieur au déplacement dudit élément de transmission 34. Dans l'exemple représenté, la seconde partie de patte 33 comporte avantageusement deux branches 33a et 33b. Ces branches sont de préférence convexes et fixées d'une part à la première partie de patte 32, et d'autre part à l'élément de transmission 34. Comme visible sur les figures 3 à 7, ces branches 33a et 33b peuvent former une structure ovoïde avec deux sommets opposés, l'un formé par ladite jonction J, l'autre formé par ledit élément de transmission 34. Un déplacement de l'élément de transmission 34 provoque donc un étirement de cette structure ovoïde, qui tire sur la première partie de patte 32. La figure 5 montre l'amplification du déplacement. L'élément de transmission 34 est déplacé en début de course d'actionnement vers le bas sur la figure 5, dans le sens de la flèche F. L'élément de transmission 34, et donc la seconde partie de patte 33, se déplace en translation, alors que la première partie de patte 32 est déplacée en rotation. Le fait que la jonction J soit décalée par rapport à l'axe de rotation de la première partie de patte 32 provoque une amplification du déplacement de la dent 35, située de l'autre côté de la jonction J par rapport à cet axe de rotation. Une projection en translation du déplacement de cette dent est représentée schématiquement sur la figure 5 et comparée au déplacement translatif « a » de l'élément de transmission 34. On constate que dans cet exemple, où la jonction est environ au centre de la première partie de patte 32, le facteur d'amplification est environ de 2. Bien entendu, en modifiant la position de la jonction J, on peut modifier le facteur d'amplification α, sachant que α sera toujours supérieur à 1. Avantageusement, la seconde partie de patte 33 peut être guidée lors de son déplacement par des moyens appropriés (non représentés), prévus par exemple dans le corps 50 du dispositif. Avantageusement, les branches 33a et 33b reviennent élastiquement vers leur position de repos après actionnement.

Bien entendu, la structure élastique à deux branches convexes pourrait être remplacée par une quelconque structure élastique monobranche ou multibranche de forme quelconque. L'essentiel est que cette structure soit fixée à la première partie de patte 32 et qu'elle soit élastiquement déformable pour d'une part provoquer le déplacement rotatif de la première partie de patte en début de course d'actionnement, et d'autre part permettre la poursuite de la course d'actionnement jusqu'à son terme.

Le nombre de dents sur la denture 19 et la forme du profil creux 18 de la roue de comptage 10 donnent les caractéristiques de l'indicateur de doses, et notamment le nombre de doses que cet indicateur peut compter. Le nombre maximal de doses et le mode d'affichage peuvent être variés à souhait en modifiant la structure du profil 18, les moyens d'indication, ou le nombre de dents sur la denture 19. La présente invention permet donc de réaliser des indicateurs de doses adaptés à compter un nombre quelconque de doses, sans modifier la géométrie ou la taille dudit indicateur. Comme déjà précisé précédemment, la structure dimensionnelle du présent indicateur est particulièrement faible, notamment dans la dimension de l'épaisseur, et cet indicateur A peut donc très facilement être intégré dans les dispositifs de distribution de produit fluide B existants, comme cela est visible sur les figures 1 et 2.

L'indicateur de doses de la présente invention permet de visualiser de manière simple, peu coûteuse et progressive le nombre de doses distribuées ou le nombre de doses restant à distribuer dans le dispositif. La structure de l'indicateur est très mince, indépendamment du nombre de doses qu'il doit indiquer, et il ne comporte aucune partie saillante impliquant une modification du dispositif auquel il est appliqué. Comme visible sur la figure 1, l'indicateur de doses A de la présente invention s'applique très facilement à tous les dispositifs existants, sans que ceux-ci ne doivent être modifiés. La présence de l'indicateur A ne modifie pas non plus le processus d'assemblage du dispositif B. L'indicateur peut par exemple être mis en place dans le dispositif B à travers une ouverture prévue à cet effet sur la partie frontale du corps 50 du dispositif. Un autre avantage du présent indicateur est que les moyens d'actionnement de l'indicateur sont intégrés dans celui-ci, de sorte que l'indicateur forme une unité autonome et séparée qui peut être pré-assemblée, et intégrée aisément dans n'importe quel dispositif de distribution de produit fluide. L'indicateur de doses de la présente invention garantit surtout l'actionnement dudit indicateur en tout début de course d'actionnement, notamment pendant la course initiale qui se produit avant le début de l'expulsion de la dose. Même lorsque cette course initiale est très courte, les moyens d'amplification de la présente invention assurent un comptage fiable.

Bien entendu, la présente invention a été décrite en référence à un mode de réalisation particulier de celle-ci, représenté sur les dessins, mais elle n'est aucunement limitée à cette forme de réalisation particulière. Au contraire, un homme du métier peut y apporter toutes modifications sans sortir du cadre de la présente invention tel que défini dans les revendications annexées.

## Revendications

1. Indicateur de doses (A) pour dispositif de distribution de produit fluide (B), comportant au moins un moyen de comptage rotatif (10) déplaçable en rotation, ledit au moins un moyen de comptage comportant des moyens d'indication (15), indiquant le nombre de doses distribuées ou restant à distribuer, ledit au moins un moyen de comptage étant actionné par un organe d'actionnement (35) lui-même actionné par un élément de transmission (34) adapté à coopérer avec une partie (54) dudit dispositif de distribution à chaque actionnement de celui-ci, ledit indicateur de doses comportant des moyens d'amplification adaptés à amplifier le déplacement dudit élément de transmission (34) à chaque actionnement, de sorte que le déplacement dudit organe d'actionnement (35) est supérieur au déplacement dudit élément de transmission (34), ledit au moins un moyen de comptage rotatif comportant une roue de comptage rotative (10) comportant une denture (19), ladite denture (19) coopérant avec des moyens d'actionnement (31, 34, 35) adaptés à faire tourner ladite roue rotative (10), **caractérisé en ce que** lesdits moyens d'actionnement comportent une patte flexible (31) comportant une première partie de patte flexible (32) et une seconde partie de patte flexible (33) plus rigide que la première partie de patte (32), la première partie de patte (32) supportant une dent d'actionnement (35) adaptée à coopérer avec la denture (19) de ladite roue de comptage rotative (10) à chaque actionnement du dispositif, et la seconde partie de patte (33) supportant l'élément de transmission (34) adapté à coopérer avec ledit dispositif de distribution de produit fluide (B) à chaque actionnement de celui-ci, la seconde partie de patte (33) étant fixée d'une part à ladite première partie de patte (32) et d'autre part audit élément de transmission (34), résultant en un déplacement amplifié de ladite dent d'actionnement (35) par rapport au déplacement dudit élément de transmission (34).

2. Indicateur selon la revendication 1, dans lequel ladite patte flexible (31) est solidaire d'une bague (30) entourant ladite denture (19), ladite patte flexible (31) venant coopérer avec ladite denture (19) chaque fois qu'une dose est distribuée.

3. Indicateur selon la revendication 2, dans lequel ladite bague (30) comporte des moyens anti-retour (36, 37), empêchant ledit disque rotatif (10) de tourner dans le sens opposé à celui induit par ladite patte flexible (31).

4. Indicateur selon la revendication 2 ou 3, dans lequel ladite bague (30) comporte une butée (39) adaptée à coopérer avec un élément de blocage (38) solidaire de ladite patte flexible (31) pour limiter la rotation de ladite roue de comptage rotative (10).

5. Indicateur selon la revendication 4, dans lequel la seconde partie de patte (33) plus rigide est adaptée à se fléchir à partir du moment où l'élément de blocage (38) est bloqué par les moyens de butée (39) de la bague (30).

6. Indicateur selon l'une quelconque des revendications précédentes, dans lequel la rotation de la roue de comptage rotative (10) est réalisée au début de la course d'actionnement du dispositif de distribution de produit fluide (B), la flexion de la seconde partie de patte (33) plus rigide permettant une poursuite de ladite course d'actionnement du dispositif de distribution de produit fluide (B) jusqu'à son terme malgré le blocage de l'élément de blocage (38) par les moyens de butée (39).

7. Indicateur selon l'une quelconque des revendications précédentes, dans lequel ledit élément de transmission (34) est un épaulement solidaire d'une patte flexible (31), et coopérant avec une partie (54) du dispositif de distribution de produit fluide (B) qui est mobile pendant l'actionnement.

8. Indicateur selon l'une quelconque des revendications précédentes, dans lequel l'indicateur (A) comporte un organe translatif (20) déplaçable en translation, les moyens d'indication (15) coopérant avec une ouverture de visualisation (25) prévue dans ledit organe translatif (20), ladite roue de comptage rotative (10) comportant un profil creux (18) coopérant avec une projection (28) dudit organe translatif (20), la forme dudit profil creux (18) étant telle qu'au moins certaines rotations de ledit au moins un moyen de comptage comportant une roue de comptage rotative (10) entraînent une translation dudit organe translatif (20), modifiant la position dudit organe translatif (20) par rapport à ladite roue de comptage (10).

9. Indicateur selon la revendication 8, dans lequel ladite roue de comptage rotative (10) et ledit organe translatif (20) sont disposés dans un couvercle (40) comportant une fenêtre de visualisation (45) coopérant avec l'ouverture de visualisation (25) de l'organe translatif (20).

10. Indicateur selon la revendication 9, dans lequel la roue de comptage rotative (10), l'organe translatif (20), les moyens d'actionnement (31, 34, 35) et le couvercle (40) forment une unité, qui peut être assemblée dans un dispositif de distribution de produit fluide (B).

11. Indicateur selon l'une quelconque des revendications 8 à 10, dans lequel lesdits moyens d'indication (15) suivent au moins partiellement ledit profil creux (18).

12. Indicateur selon l'une quelconque des revendications 8 à 11, dans lequel la forme dudit profil creux (18) est irrégulière de sorte que l'indication de dose est progressive.

13. Indicateur selon l'une quelconque des revendications 8 à 12, dans lequel ledit profil creux (18) est au moins partiellement en forme de spirale.

14. Indicateur selon l'une quelconque des revendications précédentes, dans lequel lesdits moyens d'indication (15) sont des nombres et/ou des symboles.

15. Indicateur selon l'une quelconque des revendications précédentes, dans lequel lesdits moyens d'amplification transforme un déplacement translatif (a) de l'élément de transmission (34) en un déplacement rotatif de l'organe d'actionnement (35), la projection en translation dudit déplacement rotatif étant α .a, avec α > 1.

16. Indicateur selon l'une quelconque des revendications précédentes, dans lequel ladite seconde partie de patte flexible (33) comporte une structure élastiquement déformable.

17. Indicateur selon la revendication 16, dans lequel ladite seconde partie de patte flexible (33) comporte deux branches (33a, 33b) formant une structure ovoïde ayant deux sommets opposés formés d'une part par l'élément de transmission (34) et d'autre part par la jonction (J) à la première partie de patte (32), ladite structure ovoïde pouvant être étirée par déplacement dudit élément de transmission (34) et revenant élastiquement vers sa position de repos lorsque l'élément de transmission n'est plus sollicité.

18. Dispositif de distribution de produit fluide (B) comportant un réservoir de produit (51) et un organe de distribution (52), tel qu'une pompe ou une valve, monté sur ledit réservoir (51), **caractérisé en ce qu'**il comporte un indicateur de doses (A) selon l'une quelconque des revendications précédentes.

19. Dispositif selon la revendication 18, dans lequel l'indicateur de doses (A) est actionné par une partie (54) du dispositif de distribution (B) qui est déplacée lors de l'actionnement du dispositif (B), et qui coopère avec un élément de transmission (34) dudit indicateur (A).

## Patentansprüche

1. Dosisindikator (A) für eine Ausgabevorrichtung für ein fluides Produkt (B), aufweisend mindestens ein drehbares Zählmittel (10), das drehverschiebbar ist, wobei das mindestens eine Zählmittel Anzeigemittel (15) aufweist, die die Anzahl an ausgegebenen oder noch auszugebenden Dosen anzeigt, wobei das mindestens eine Zählmittel durch ein Betätigungselement (35) betätigt wird, welches selber durch ein Übertragungselement (34) betätigt wird, welches dazu geeignet ist, bei jeder Betätigung von diesem mit einem Teil (54) der Ausgabevorrichtung zusammenzuwirken, wobei der Dosisindikator Verstärkungsmittel umfasst, die dazu geeignet sind, die Verschiebung des Übertragungselements (34) bei jeder Betätigung zu verstärken, so dass die Verschiebung des Betätigungselementes (35) größer ist als die Verschiebung des Übertragungselementes (34), wobei das mindestens eine drehbare Zählmittel ein drehbares Zählrad (10) aufweist, welches eine Verzahnung (19) aufweist, wobei die Verzahnung (19) mit den Betätigungsmitteln (31, 34, 35) zusammenwirkt, die dazu geeignet sind, das drehbare Rad (10) zu drehen, **dadurch gekennzeichnet, dass** die Betätigungsmittel eine flexible Lasche (31) aufweisen, die einen ersten flexiblen Laschenteil (32) und einen zweiten flexiblen Laschenteil (33), der starrer ist als der erste Laschenteil (32), aufweist, wobei der erste Laschenteil (32) einen Betätigungszahn (35) trägt, der dazu geeignet ist, bei jeder Betätigung der Vorrichtung mit der Verzahnung (19) des drehbaren Zählrads (10) zusammenzuwirken, und der zweite Laschenteil (33) das Übertragungselement (34) trägt, das dazu geeignet ist, bei jeder Betätigung der Ausgabevorrichtung für fluides Produkt (B) mit dieser zusammenzuwirken, wobei der zweite Laschenteil (33) einerseits an dem ersten Laschenteil (32) und andererseits an dem Übertragungselement (34) befestigt ist, was zu einer verstärkten Verschiebung des Betätigungszahns (35) in Bezug auf die Verschiebung des Übertragungselements (34) führt.

2. Indikator nach Anspruch 1, wobei die flexible Lasche (31) mit einem Ring (30) einstückig gebildet ist, der die Verzahnung (19) umgibt, wobei die flexible Lasche (31) jedes Mal mit der Verzahnung (19) zusammenwirkt, wenn eine Dosis ausgegeben wird.

3. Indikator nach Anspruch 2, wobei der Ring (30) Rückführsperrmittel (36, 37) aufweist, die verhindern, dass sich die Drehscheibe (10) in eine Richtung entgegengesetzt zu der von der flexiblen Lasche (31) induzierten Richtung dreht.

4. Indikator nach Anspruch 2 oder 3, wobei der Ring (30) einen Anschlag (39) aufweist, der dazu geeignet ist, mit einem Sperrelement (38) zusammenzuwirken, das in einem Stück mit der flexiblen Lasche (31) ausgebildet ist, um die Drehung des drehbaren Zählrads (10) zu begrenzen.

5. Indikator nach Anspruch 4, wobei der zweite, steifere Laschenteil (33) dazu geeignet ist, ab dem Moment durchzubiegen, ab dem das Sperrelement (38) durch die Anschlagmittel (39) des Rings (30) blockiert ist.

6. Indikator nach einem der vorhergehenden Ansprüche, wobei die Drehung des drehbaren Zählrads (10) zu Beginn des Betätigungshubs der Ausgabevorrichtung für ein fluides Produkt (B) realisiert wird, wobei das Durchbiegen des zweiten, steiferen Laschenteils (33) eine Weiterführung des Betätigungshubs der Ausgabevorrichtung für ein fluides Produkt (B) bis zum Ende trotz der Sperrung des Sperrelements (38) durch die Anschlagmittel (39) erlaubt.

7. Indikator nach einem der vorhergehenden Ansprüche, wobei das Übertragungselement (34) ein mit einer flexiblen Lasche (31) in einem Stück ausgebildeter Absatz ist und mit einem Teil (54) der Ausgabevorrichtung für ein fluides Produkt (B) zusammenwirkt, das während der Betätigung beweglich ist.

8. Indikator nach einem der vorhergehenden Ansprüche, wobei der Indikator (A) eine Verschiebeeinrichtung (20) aufweist, die verschiebbar ist, wobei die Anzeigemittel (15) mit einer Sichtöffnung (25) zusammenwirken, die in der Verschiebeeinrichtung (20) vorgesehen ist, wobei das drehbare Zählrad (10) ein Hohlprofil (18) aufweist, das mit einem Vorsprung (28) der Verschiebeeinrichtung (20) zusammenwirkt, wobei die Form des Hohlprofils (18) derart ist, dass mindestens bestimmte Drehungen des mindestens einen Zählmittels, das ein drehbares Zählrad (10) aufweist, eine Verschiebung der Verschiebeeinrichtung (20) nach sich ziehen, die die Position der Verschiebeeinrichtung (20) in Bezug auf das Zählrad (10) ändert.

9. Indikator nach Anspruch 8, wobei das drehbare Zählrad (10) und die Verschiebeeinrichtung (20) in einem Deckel (40) angeordnet sind, der ein Sichtfenster (45) aufweist, das mit der Sichtöffnung (25) der Verschiebeeinrichtung (20) zusammenwirkt.

10. Indikator nach Anspruch 9, wobei das drehbare Zählrad (10), die Verschiebeeinrichtung (20), die Betätigungsmittel (31, 34, 35) und der Deckel (40) eine Einheit bilden, die in eine Ausgabevorrichtung für ein fluides Produkt (B) montiert werden kann.

11. Indikator nach einem der Ansprüche 8 bis 10, wobei die Anzeigemittel (15) zumindest teilweise dem Hohlprofil (18) folgen.

12. Indikator nach einem der Ansprüche 8 bis 11, wobei die Form des Hohlprofils (18) ungleichmäßig ist, so dass der Dosisindikator progressiv ist.

13. Indikator nach einem der Ansprüche 8 bis 12, wobei das Hohlprofil (18) zumindest teilweise spiralförmig ist.

14. Indikator nach einem der vorhergehenden Ansprüche, wobei die Anzeigemittel (15) Zahlen und/oder Symbole sind.

15. Indikator nach einem der vorhergehenden Ansprüche, wobei die Verstärkungsmittel eine Translationsverschiebung (a) des Übertragungselements (34) in eine Drehverschiebung des Betätigungselements (35) umwandeln, wobei die Translationsprojektion der Drehverschiebung α .a ist, wobei α > 1.

16. Indikator nach einem der vorhergehenden Ansprüche, wobei der zweite flexible Laschenteil (33) eine elastisch verformbare Struktur aufweist.

17. Indikator nach Anspruch 16, wobei der zweite flexible Laschenteil (33) zwei Schenkel (33a, 33b) aufweist, die eine ovale Struktur mit zwei entgegengesetzten Spitzen bilden, die einerseits von dem Übertragungselement (34) und andererseits von der Verbindungsstelle (J) an dem ersten Laschenteil (32) gebildet sind, wobei die ovale Struktur durch Verschiebung des Übertragungselementes (34) auseinandergezogen werden kann und elastisch in ihre Ruheposition zurückkehrt, wenn das Übertragungselement nicht mehr vorgespannt ist.

18. Ausgabevorrichtung für ein fluides Produkt (B), aufweisend einen Produktbehälter (51) und eine Ausgabeeinrichtung (52), wie eine Pumpe oder ein Ventil, die auf dem Behälter (51) montiert ist, **dadurch gekennzeichnet, dass** sie einen Dosisindikator (A) nach einem der vorhergehenden Ansprüche aufweist.

19. Vorrichtung nach Anspruch 18, wobei der Dosisindikator (A) durch ein Teil (54) der Ausgabevorrichtung (B) betätigt wird, welches bei Betätigung der Vorrichtung (B) verschoben wird und mit einem Übertragungselement (34) des Indikators (A) zusammenwirkt.

## Claims

1. Dose indicator (A) for fluid product dispensing device (B) comprising at least one rotary counting means (10) which can be moved in rotation, said at least one counting means comprising indication means (15) indicating the number of doses dispensed or remaining to be dispensed, said at least one counting means being actuated by an actuating member (35) itself actuated by a transmission element (34) adapted to cooperate with one part (54) of said dispensing device on each actuation thereof, said dose indicator comprising amplification means adapted to amplify the movement of said transmission element (34) on each actuation, so that the movement of said actuating member (35) is greater than the movement of said transmission element (34), said at least one rotary counting means comprising a rotary counting wheel (10) comprising cogging (19), said cogging (19) cooperating with actuating means (31,34,35) adapted to cause said rotary wheel (10) to rotate, **characterized in that** said actuating means comprise a flexible lug (31) comprising a first flexible lug part (32) and a second flexible lug part (33) more rigid than the first lug part (32), the first lug part (32) bearing an actuating tooth (35) adapted to cooperate with cogging (19) of said rotary counting wheel (10) on each actuation of the device, the second lug part (33) bearing the transmission element (34) adapted to cooperate with said fluid product dispensing device (B) whenever it is actuated, the second lug part (33) being attached firstly to said first lug part (32) and secondly to said transmission element (34) resulting in an amplified movement of said actuating tooth (35) with respect to the movement of said transmission element (34).

2. Indicator as in claim 1, wherein said flexible lug (31) is joined to a ring (30) surrounding said cogging (19), said flexible lug (31) coming to cooperate with said cogging (19) whenever a dose is dispensed.

3. Indicator as in claim 2, wherein said ring (30) comprises anti-reverse means (36, 37) preventing said rotary disc (10) from rotating in the opposite direction to the direction induced by said flexible lug (31).

4. Indicator as in claim 2 or 3, wherein said ring (30) comprises an abutment (39) adapted to cooperate with a locking element (38) joined to said flexible lug (31) to limit the rotation of said rotary counting wheel (10).

5. Indicator as in claim 4, wherein the second, more rigid lug part (33) is adapted so that it deflects on and after the time the locking element (38) is locked by the abutment means (39) of the ring (30).

6. Indicator as in any of the preceding claims, wherein the rotation of the rotary counting wheel (10) occurs at the start of the actuation distance of the fluid product dispensing device (B), the flexion of the second, more rigid lug part (33) enabling said actuation distance of the fluid product dispensing device (B) to be completed up to its end despite locking of the locking element (38) by the abutment means (39).

7. Indicator as in any of the preceding claims, wherein said transmission element (34) is a shoulder joined to a flexible lug (31) and cooperating with one part (54) of the fluid product dispensing device (B) which is mobile during actuation.

8. Indicator as in any of the preceding claims, wherein the indicator (A) comprises a translatable member (20) which can be moved in translation, the indication means (15) cooperating with a display opening (25) provided in said translatable member (20), said rotary counting wheel (10) comprising a hollow profile (18) cooperating with a projection (28) of said translatable member (20), the shape of said hollow profile (18) being such that at least some rotations of said at least one counting means comprising a rotary counting wheel (10) give rise to translation of said translatable member (20), modifying the position of said translatable member (20) with respect to said counting wheel (10).

9. Indicator as in claim 8, wherein said rotary counting wheel (10) and said translatable member (20) are arranged in a lid (40) comprising a display window (45) cooperating with the display opening (25) of the translatable member (20).

10. Indicator as in claim 9, wherein the rotary counting wheel (10), the translatable member (20), the actuating means (31, 34, 35) and the lid (40) form a unit which can be assembled in a fluid product dispensing device (B).

11. Indicator as in any of claims 8 to 10, wherein said indication means (15) follow said hollow profile (18) at least in part.

12. Indicator as in any of claims 8 to 11, wherein the shape of said hollow profile (18) is irregular so that dose indication is progressive.

13. Indicator as in any of claims 8 to 12, wherein said hollow profile (18) is at least partly of spiral shape.

14. Indicator as in any of the preceding claims, wherein said indication means (15) are numbers and/or symbols.

15. Indicator as in any of the preceding claims, wherein said amplification means transform a translation movement (a) of the transmission element (34) into a rotary movement of the actuating member (35), the translation projection of said rotary movement being α.a, where α > 1.

16. Indicator as in any of the preceding claims, wherein said second flexible lug part (33) comprises an elastically deformable structure.

17. Indicator as in claim 16, wherein said second flexible lug part (33) comprises two branches (33a, 33b) forming an ovoid structure having two opposite apexes formed firstly by the transmission element (34) and secondly by the junction (J) with the first lug part (32), said ovoid structure able to be stretched by movement of said transmission element (34) and returning elastically to its rest position the transmission element is not urged anymore.

18. Fluid product dispensing device (B) comprising a product reservoir (51) and a dispensing member (52) such as a pump or valve mounted on said reservoir (51), **characterized in that** it comprises a dose indicator (A) as in any of the preceding claims.

19. Device as in claim 18, wherein the dose indicator (A) is actuated by one part (54) of the dispensing device (B) which is moved during actuation of device (B) and which cooperates with a transmission element (34) of said indicator (A).
